# EUROPEAN PATENT APPLICATION

(11) **EP 1 004 590 A2**
(43) Date of publication of application: **31.05.2000**
(21) Application number: 99120748.1
(22) Date of filing: 20.10.1999
(51) Int. Cl.: C07H 13/04, A61K 31/70, A61P 33/00

(54) **Glucidic esters of branched carboxylic acids capable of inducing erythroid cellular differentiation**

(30) Priority: 22.10.1998 IT TO980899
(71) Applicant: ASSOCIAZIONE VENETA PER LA LOTTA ALLA TALASSEMIA, 45100 ROVIGO (IT); ASSOCIAZIONE PER LA LOTTA ALLA TALASSEMIA DI FERRARA, 44100 Ferrara (IT); CHIESI FARMACEUTICI S.p.A., I-43100 Parma (IT)
(72) Inventor: Bergonzi, Maria Camilla, 52100 Arezzo (IT); Bianchi, Nicoletta, 44020 Mezzogoro (Ferrara) (IT); Catelani, Giorgio, 56021 Cascina (Pisa) (IT); D'Andrea, Felicia, 56011 Calci (Pisa) (IT); Gambari, Roberto, 40033 Casalecchio Di Reno (Bologna) (IT); Lipucci Di Paola, Michele, 56123 Pisa (IT); Osti, Fabio, 46020 Villa Poma (Mantova) (IT)
(74) Representative: Rambelli, Paolo

(57) **Abstract**

The present invention provides compounds which are esters of a carbohydrate, constituted by a carbohydrate unit or a glycoside derived therefrom, having at least one alcohol group esterified with a branched chain carboxylic acid and having the remaining alcohol groups in part protected with a protector group, such as an isopropylidene group, and partly in the form of free hydroxyls. These carbohydrate esters being provided with a significant biological activity as erythroid cellular differentiation inducers can be utilised for the preparation of a medicament for the therapeutic treatment of β-talaxemia or tumours.

## Description

### Technical field

The present invention relates to compounds which are carbohydrate esters of branched chain carboxylic acids capable of inducing erythroid cellular differentiation, to the use of said compounds for the preparation of a medicament for the therapeutic treatment of β-talaxemia, to the use of the said compounds for the preparation of a medicament for the therapeutic treatment of tumours, and a pharmaceutical composition comprising at least one of the said compounds and a pharmaceutically acceptable carrier.

### State of the prior art

The existence of substances, such as 5-azacytidine, hydroxyurea, erythropoietin, able to induce biosynthesis of haemoglobin-F (foetal) in adult subjects is known from the prior art (see in this respect De Simone J. et al., Proc. Natl. Acad. Sci. USA 79:4428-4431, 1982 and Rochette J. et al., Blood Reviews 8, 213-224, 1994).

Such substances, here indicated as "modifiers of the biological response", are able to activate the process of transcription of γ-globin genes and therefore induce erythroid cellular differentiation. The activation of the transcription of genes for γ-globin makes the production of haemoglobin-F possible, thus mimicking the HPFH phenotype (High Persistence of Foetal Haemoglobin) which confers a favourable clinical outlook even on patients affected by β-talaxemia in homozygote form.

It is likewise known that some linear chain carboxylic acids act as modifiers of the biological response, stimulating the synthesis of foetal haemoglobin, in particular by activating the transcription of genes for the γ-globin (Perrine SP et al., N. Engl. J. Med. 328:81-86, 1993 and Torkelson S. et al., Blood Cells, Molecules and Diseases, 22:150-158, 1996). These carboxylic acids are moreover able to inhibit the growth and induce the differentiation of malignant and pre-malignant cells, particularly leukemic cells, therefore acting as antitumour agents.

The said linear carboxylic acids are, however, characterised by a very short plasma lifetime *in vivo,* which can be extended by esterification with the alcohol group of a carbohydrate (Pouillart P. et al., J. Pharm. Sciences 81: 241-224, 1992).

US 5185436 describes esters of n-butyric acid with polyhydroxylated compounds selected from the group consisting of polyhydric alcohols, aldose or ketose monosaccharides in cyclic or linear form and acetonides derived therefrom.

WO 96/03411 describes carbohydrates and their derivatives, among which D-mannose or a pentitol such as xylithol are particularly preferred, esterified with a linear carboxylic acid selected from the group consisting of phenylacetic acid, 3-phenylpropionic acid, 4-phenylbutyric acid and n-butyric acid.

The object of the present invention is to provide new compounds derived from branched chain carboxylic acids having a greater biological activity as inducers of erythroid cellular differentiation than those of the previously described compounds.

### Description of the Invention

The subject of the present invention comprises esters of carbohydrates constituted by a carbohydrate unit or a glycoside derived therefrom, in which at least one alcohol group is esterified with a branched chain carboxylic acid, whilst the remaining alcohol groups are in part protected with a protecting group and are in part in the form of free hydroxyls.

In a preferred embodiment of the present invention the said carbohydrate unit is D-glucofuranose, D-galactopyranose, D-mannose, saccharose or lactose.

In another preferred embodiment of the present invention the said protecting group is isopropylidene.

In another preferred embodiment of the present invention the said protecting group occupies positions 1,2 or 3,4 of the said carbohydrate unit.

In a further preferred embodiment of the present invention the said branched chain carboxylic acids are selected from the group comprising 2-methylpropionic or isobutyric acid (HCO₂CH(CH₃)₂), 2,2-dimethylpropionic or pivaloic acid (HCO₂C(CH₃)₃), 3,3-diphenylpropionic acid (HCO₂CH₂CH(C₆H₅)₂), 3-methylbutyric acid (HCO₂CH₂CH(CH₃)₂), 3-phenylbutyric acid (HCO₂CH₂CH(CH₃)C₆H₅), 3,3-dimethylbutyric acid (HCO₂CH₂C(CH₃)₃), 3-methylpentanoic acid (HCO₂CH₂CH(CH₃)CH₂CH₃).

In a more preferred embodiment of the present invention the partially protected carbohydrate unit from which the esters forming the subject of the present invention are derived corresponds to the following structures, and the alcohol groups protected with isopropylidene groups are those specifically indicated for each carbohydrate unit: the hydroxyl group 3-OH of 1,2-O-isopropylidene D-glucofuranose, the hydroxyl group 6-OH of methyl. 3,4-O-isopropylidene-β-D-galactopyranoside, the hydroxyl group 2-OH of methyl 3,4-O-isopropylidene-β-D-galactopyranoside, the hydroxyl group 6-OH of methyl 3,4-O-isopropylidene-α-D-galactopyranoside, the hydroxyl group 2-OH of methyl 3,4-O-isopropylidene-α-D-galactopyranoside.

Also the subject of the present invention are other esters of branched chain acids constituted by residues of the above listed carboxylic acids and other types of carbohydrate units deriving from common carbohydrates, as long as they are characterised by having at least one specific alcohol group of the carbohydrate residue esterified with a branched chain carboxylic acid, with the remaining alcohol groups in part protected as isopropylidene groups and in part in the form of free hydroxyls.

The subject of the present invention also includes the previously described compounds for use as inducers of the erythroid cellular differentiation, possibly in combination with at least one other modifier of the biological response preferably selected from the group consisting of cytosine arabinoside, retinoic acid, plicamycin, mithramycin, hydroxyurea, guanine, guanosine triphosphate (GTP), guanosine diphosphate (GDP) and guanosine monophosphate (GMP), although most preferably cytosine arabinoside or retinoic acid.

Also the subject of the present invention is the use of the previously described compounds, possibly in combination with at least one other modifier of the biological response as previously defined, for the preparation of a medicament for the therapeutic treatment of β-talaxemia or tumours.

Still a subject of the present invention is a pharmaceutical composition comprising at least one of the previously described compounds and a pharmaceutically acceptable carrier, possibly in combination with at least one other modifier of the biological response as previously defined.

The advantages provided by the use of the compounds forming the subject of the invention as erythroid cellular differentiation inducers have been evaluated by means of a comparative study the results of which are schematically indicated in tables 1, 2 and 3. The object of this study was that of comparing the level of erythroid differentiation obtained by treating the human cell line K562, independently, with two compounds falling within the scope of the present invention (that is to say 3-O-isobutyryl-1,2-O-isopropylidene-D-glucofuranose, indicated as GG6B and 3-O-pivaloyl-1,2-O-isopropylidene-D-glucofuranose, indicated as GG7B) and two reference compounds (that is to say n-butyric acid and 3-O-n-butanoyl-1,2-O-isopropylidene-α-D-glucofuranose, indicated as GG3B).

The compounds forming the subject of the invention are prepared according to a general scheme which envisages the following three general phases:
1) Preparation of a selected carboydrate derivative having at least one free alcohol function and the others protected by protecting groups. The preferred protecting groups bond to the said carbohydrate unit giving rise to acetal bridges having the following general formula: 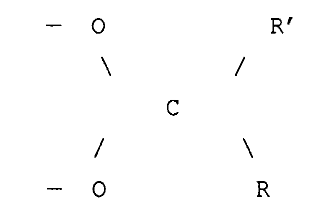 in which the R-C-R' group preferably derives from phenylmethylene, methylene, cyclohexylidene and, more preferably, isopropylidene.
2) Esterification of the free alcohol functions of the carbohydrate by treatment with the acid chloride of a branched chain carboxylic acid.
3) Selective removal of the said protecting groups in such a way as to conserve a protecting group and the ester group of the branched chain acid.

The methods of synthesis to be utilised to perform the three above-described general phases are chosen from among the numerous commonly utilised protection and deprotection techniques for alcohol groups of carbohydrates as described in the preceding literature, for example in "Methods in Carbohydrate Chemistry", Academic Press, Volumes 2, 6 and 8.

The following examples are presented for the purpose of illustration and are not intended to limit in any way the scope of the present invention.

### Examples

### Preparation of carbohydrate esters

### Example 1

Preparation of 3-O-isobutyryl-1,2-O-isopropylidene-D-glucofuranose with methods similar to those reported by P. Y Goueth and collaborators, Journal Carbohydrate Chemistry 13 (1994) 249. To a commercial solution of 1,2:5,6-di-O-isopropylidene-D-glucofuranose (Aldrich) (5.0g, 19.21 mmol) previously dried in a drying pistol (3 hours at 50°C) in anhyrous toluene (45ml), 6.4ml (46.1 mmol) of anhydrous triethylamine and 4.3 mmols of 2-methylpropanoyl chloride are added and the mixture is left under magnetic stirring at ambient temperature. After 24 hours the reaction mixture is filtered on a porous septum to eliminate the triethyl ammonium chloride which forms, then concentrated to a small volume, added to a saturated solution of NaHCO₃ (10ml) and left under magnetic stirring for 30 minutes at ambient temperature (pH about 8). The resultant suspension is extracted with ethyl acetate (AcOEt) (4x20ml), the organic phases are dried with MgSO₄, the solvent is filtered and evaporated at reduced pressure. The crude product obtained (6.11g) is purified by means of flash-chromatography on a silica gel column, eluted with hexane/AcOEt 9:1, to give pure 3-O-(2-methylpropanoyl)-1, 2:5,6-di-O-isopropylidene-D-glucofuranose (5.70g) with a 91% yield, as a crystalline solid with Rf=0.47 (hexane/AcOEt 1:1); [α]_{D} = -27.37° (c=1.03, CH₂Cl₂); m.p. =33-35°C. The compound thus prepared (5.70g) was suspended in 250ml of aqueous AcOH at 80% and maintained under agitation at 60°C. After 6 hours and 30 minutes the solution is cooled to ambient temperature, added to 250ml of toluene and evaporated at reduced pressure, repeating this operation twice. The crude product obtained (4.99g), of solid consistency, is purified by crystallisation with toluene/hexane obtaining the pure 3-O-isobutyryl-1,2-O-isopropylidene-D-glucofuranose (4.43g) with a 91% yield as a crystalline solid having Rf=0.15 (hexane/AcOEt 1:1); [α]_{D} = +16.38° (c=1.19, CHCl₃); m.p.=78-81°C; elemental analysis: (calculated for C₁₃H₂₂O₇: C 53.78%; H 7.63%) C 53.59%; H 7.66%; ¹H-NMR (CDCl₃): δ (ppm) 5.90 (d, 1 H, J_{1,2}= 3.7 Hz, H-1), 4.44 (dd, 1H, J_{2,3} = 2.6Hz, H-2), 5.25 (dd, H-3), 4.19 (dd, H-4), 3.62-3.83 (m, 3H, H-5 + 2-H-6), 2.63(m, 1 H, CH group of isobutyryl), 1.32 and 1.52 (2 s, 6H, methyl groups of isopropylidene), 1.20 (dd, 6 H, methyl groups of isobutyryl); ¹³C-NMR: δ (ppm) 104.81 (C-1), 83.03 (C-2), 76.36 (C-3), 79.47 (C-4), 68.08 (C-5), 64.11 (C-6), 26.57 and 26.15 (methyl groups of isopropylidene) 112.34 (C of acetal), 177.40 (C=O), 33.96, 18.92, 18.82 (C of isobutyryl).

### Example 2

Preparation of 3-O-pivaloyl-1,2-O-isopropylidene-D-glucofuranose in a manner similar to the process described in Example 1.

To a solution of commercial 1,2:5,6-di-O-isopropylidene-D-glucofuranose (3.0g, 11.52 mmol) in anhydrous toluene (30 ml), 3.85 ml of anhydrous triethylamine and 2.7 mmol of the chloride of 2,2-dimethylpropanoic acid are added. The solution is warmed to 100°C for 11 hours, then cooled, filtered and concentrated to dryness. The crude residue (4.31g) is purified by flash chromatography on a silica gel column, eluted with hexane/AcOEt, 9:1 to give pure 3-O-(2,2-dimethylpropanoyl)-1,2:5,6-di-O-isopropylidene-D-glucofuranose (2.12g) with a yield of 53%, as a wax solid with Rf = 0.65 (hexane/AcOEt 1:1); [α]_{D} = -30.4° (c = 1.5, CHCl₃); m.p. = 33-35°C. A portion of the thus-prepared compound (1.06g, 3.09mmol) was suspended in 45 ml of aqueous AcOH at 80% and maintained under agitation at 60°C. After 33 hours the solution is cooled to ambient temperature, added to 50ml of toluene and evaporated at reduced pressure, repeating this operation twice. The crude product obtained (1.06g) is purified by flash-chromatography on silica gel, eluted with 1:1 hexane/ethylacetate to obtain pure 3-O-pivaloyl-1,2-O-isopropylidene-D-glucofuranose (0.53g) with a 56% yield as a colourless syrup having Rf=0.46 (1:1 hexane/AcOEt); [α]_{D} = +15.4° (c = 0.9, CHCl₃); elemental analysis: (calculated for C₁₄H₂₄O₇: C 55.25%; H 7.95%); C 54.95%; H 7.96%; ¹H-NMR (CDCl₃) : δ (ppm) 5.9 (d, 1 H, J_{1,2,} = 3.7 Hz, H-1), 4.51 (dd, 1H, J_{2,3} = 2.6 Hz, H-2), 5.24 (dd, H-3), 4.20 (dd, H-4), 3.60-3.82 (m, 3H, H-5 + 2-H-6), 1.32 and 1.52 (2 s, 6H, methyl groups of isopropylidene), 1.22 (s, 9 H, methyl groups of pivaloyl); ¹³C-NMR: δ (ppm) 104.79 (C-1), 82.99 (C-2), 76.32 (C-3), 79.39 (C-4), 68.11 (C-5), 64.06 (C-6), 26.14 and 26.55 (methyl groups of isopropylidene) 112.32 (C of acetal), 178.79 (C=0), 39.02 e 27.03 (C of pivaloyl).

### Example 3

Preparation of methyl 2-O-isobutyryl-3, 4-O-isopropylidene-beta-D-galactopyranoside with a procedure similar to that reported by P.L. Barili, G. Berti, G. Catelani, F. Colonna, A. Marra, Tetrahedron Lett., 27, 2307, (1986). 5.0g (25.79 mmol) of commercial methyl β-D-galactopyranoside (Aldrich) and 160mg of para-toluenesulphonic acid are suspended in 270ml of 2,2-dimethoxypropane. The suspension is maintained under magnetic stirring at ambient temperature for 48 hours; the solution is treated with 1.0ml of triethylamine (Et₃N), the solvent is evaporated at reduced pressure (bath temperature 30°C), co-evaporating with toluene (3 x 100ml) several times. The crude reaction product (500 mg) constituted by more than 95% of methyl 3,4-O-isopropylidene-6-O-methoxyisopropyl-β-D-galactopyranoside was solubilised in 6ml of anhydrous toluene and 0.544ml (3.90mmol) of anhydrous triethylamine and 0.372ml (3.58mmol) of 2-methylpropanoyl chloride are added therein. The reaction mixture is left under magnetic stirring at ambient temperature for 46 hours. The suspension obtained is filtered on a porous septum to separate the triethylammonium salts formed during the reaction, the solvent evaporated at reduced pressure and the crude product (615mg), mainly constituted by methyl 2-O-isobutyryl-3,4-O-isopropylidene-6-O-methoxyisopropyl-β-D-galactopyranoside is solubilised in 10ml of CH₃OH, to which 14.5mg (0.057mmol) of pyridinium tosylate were added and the solution obtained is maintained under magnetic stirring at ambient temperature. After 20 minutes solid Na₂CO₃ is added, stirring is continued for 15 minutes, then the solvent is evaporated at reduced pressure to give a crude product (530mg) which, by flash chromatography on a silica gel column (1:1 petroleum ether/AcOEt) provides the pure methyl 2-O-isobutyryl-3,4-O-isopropylidene-β-D-galactopyranoside (322mg) with a yield of 65%. The product is a white solid and has: Rf=0.25 (petroleum ether/AcOEt 1:1); m.p. = 74-75°C (crystallised from hexane); [α]_{D}= + 17.4° (c = 1.06, CHCl₃); elemental analysis: (calculated for C₁₄H₂₄O₇: C 55.25%; H 7.95%) C 55.43%; H 7.74%. ¹H-NMR (CDCl₃) δ (ppm): 1.17-1.20 (d, 6H, 2CH₃, (CH₃)₂-CH); 1.34 and 1.56 (2s, 6H, 2CH₃ of dioxolane); 2.31 (s, 1H, OH); 2.55-2.62 (d.q., 1H, CHCO); 3.49 (s, 3H, OCH₃); from 3.83 to 4.02 (m, 3H, H-5, H-6 and H-6'); 4.18 (m, 2H, H-3 and H-4); 4.28 (d, 1H, J_{1,2}= 8.18 Hz, H-1); 4.98 (m, 1H, H-2). ¹³C-NMR (CDCl₃) δ (ppm): 18.58 (CH₃-CH); 18.87 (CH₃-CH); 26.27 and 27.50 (2CH₃ of dioxolane); 33.82 (CH-CO); 56.64 (OCH₃); 62.26 (C-6); 72.38, 73.11 and 73.82 (C-5), C-2 and C-4); 77.02 (C-3); 101.48 (C-1); 110.61 (quaternary C of dioxolane); 172.70 (C=0).

### Example 4

Preparation of methyl 2-O-pivaloyl-3,4-O-isopropylidene-β-D-galactopyranoside by means of esterification effected according to a procedure similar to that described in example 3, using 0.441ml (3.50 mmols) of the chloride of 2,2-dimethylpropanoic acid. After seven days, whilst the starting product is still present, the reaction mixture is subjected to a work-up similar to that of example 3. The crude product (710mg) is purified by chromatography on a silica gel column (70-230 mesh), eluted with petroleum ether/AcOEt 1:1. 384mg of slightly impure methyl 2-O-pivaloyl-3,4-O-isopropylidene-6-O-methoxyisopropyl-β-D-galactopyranoside is obtained. This is solubilised in 11ml of MEOH/H₂O 10:1, added to 0.1ml of 60% AcOH and the solution obtained is warmed under reflux. After 15 minutes 0.5ml of Et₃N is added and the solvent evaporated at reduced pressure to give a crude product (350mg) which is purified by chromotography on a silica gel column (70-230 mesh) eluted with 1:1 hexane/AcOEt. Methyl 2-O-pivaloyl-3,4-O-isopropylidene-β-D-galactopyranoside (302mg) is obtained which has: Rf = 0.25 (hexane/AcOEt 1:1); m.p. = 85-87°C (crystallised from petroleum ether); [α]_{D} = +19.3° (c = 1.07, CHCl₃); elemental analysis: (calculated for C₁₅H₂₆O₇: C 56.59%; H 8.23%); C59.69%; H 8.58%. ¹H-NMR (CDCl₃): δ (ppm) 1.22 (s, 9H, 3 CH₃, (CH₃) ₃-C; 1.34 and 1.56 (2 s, 6H, 2 CH₃ of dioxolane); 1.35 (s1 H, 1 OH); 3.48 (s, 3H, OCH₃,); from 3.83 to 4.02 (m, 3H, H-5, H-6 and H-6'); 4.20 (m, 2H, H-3 and H-4); 4.98 (d, 1 H, J_{1,2} = 8, 18 Hz, H-1); 4.98 (m, 1H, H-2). ¹³C-NMR (CDCl₃) δ (ppm): 26.92 ((CH₃)₃-C) 26.27 and 27.48 (2 CH₃ of dioxolane); 38.62 (CCO); 56.69 (OCH₃); 62.27 (C-6); 72.38, 73.10 and 73.76 (C-5, C-2 and C-4); 76.99 (C-3); 101.63 (C-1); 110.58 (quaternary C of dioxolane); 177.02 (C=O).

### Example 5

Preparation of methyl 6-O-isobutyryl-3,4-O-isopropylidene-β-D-galactopyranoside with a procedure similar to that reported by P.L Barili, G. Berti, G. Catelani, F. Colonna, A. Marra, Tetrahedron Lett., 27, 2307, (1986). Methyl-3,4-O-isopropylidene-6-O-methoxyisopropyl-β-D-galactopyranoside was prepared. To a solution of this latter crude product (1.0 g, 3.24 mmol) in 9ml of aqueous THF (0.5%) is added 34.2mg (0.129mmol) of 18 crown-6,530mg (9.4mmol) of solid KOH and 0.77ml (6.50mmol) of benzyl bromide. The suspension is left under magnetic stirring at ambient temperature for 18 hours. Then 20ml of MeOH is added, agitation continued for 30 minutes at ambient temperature and the solvent evaporated at reduced pressure to give a residue which is recovered with CH₂Cl₂ and washed with H₂O (4x20ml). The organic phase is dried with MgSO₄, then filtered and evaporated at reduced pressure to provide a crude product (1.73g) which is purified by flash-chromatography on silica gel column eluting with hexane /AcOEt 8.5:1.5 and then with hexane/AcOEt 8:2 to give 938mg (2.35mmol, 92% yield) of pure methyl 2-O-benzyl-3,4-O-isopropylidene-6-O-methoxyisopropyl-β-D-galactopyranoside which is subjected to an hydrolysis reaction according to the procedure reported in example 4. The crude product (819mg), purified by chromatography on silica gel column (70-230 mesh), provides 541mg of pure methyl 2-O-benzyl-3,4-O-isopropylidene-β-D-galactopyranoside (71% yield). A portion of the thus-prepared compound (330mg, 1.017mmol) was subjected to an esterification reaction by a procedure similar to that described in example 3 solubilising it in 6ml of anhydrous toluene and adding 0.340ml (2.44mmol) of anhydrous triethylamine (TEA) and 0.23ml (3.5mmol) of isobutanoyl chloride. After 40 minutes the suspension is treated in a manner similar to that previously described and provides 407mg of a crude product (1.032mmol) constituted exclusively by methyl 2-O-benzyl-6-O-isobutanoyl-3, 4-O-isopropylidene-β-D-galactopyranoside (quantitative yield). This is solubilised in 8ml of ethyl acetate, added to 80mg of palladium hydroxide activated on carbon at 10% (Aldrich) and left under magnetic stirring in the presence of hydrogen. After 5 hours and 30 minutes the suspension is filtered on a porous septum covered in celite, and the solvent evaporated at reduced pressure to obtain a solid crude product (319mg) from which, by crystallisation from petroleum ether, is obtained, as a white crystalline solid, a quantitative yield of methyl 6-O-isobutanoyl-3,4-O-isopropylidene-β-D-galactopyranoside. The product has: Rf = 0.20 (hexane/AcOEt 4:6); m.p. = 104-107°C (crystallised from petroleum ether); [α]_{D} = +21.7° (c = 1.12, CHCl₃); elemental analysis: (calculated for C₁₄H₂₇O₇: C 55.25%; H 7.95%) C 55.62%: H 8.05%. ¹H-NMR (CDCl₃) δ (ppm): 1.17-1.20 (d, 6H, 2CH₃, (CH₃)₂-CH); 1.34 and 1.51 (2ₛ, 6H, 2CH₃ of dioxolane); 2.56-2.63 (d.q., 1H, CHCO); 2.71 (1H, OH); 3.54 (s, 3H, OCH₃); from 3.50 to 3.58 (m, 1H, H-5): 4.00-4.16 (m, 4H, H-1, H-2, H-3, H-4); 4.37 and 4.38 (d, 2H, H-6, H-6'). ¹³C-NMR (CDCl₃) δ (ppm): 18.84 (CH₃-CH); 26.20 and 27.99 (2 CH₃ of dioxolane); 33.85 (CH-CO); 56.84 (OCH₃); 63.02 (C-6); 70.90-73.48 (C-2 C-4 and C-5); 78.68 (C-3); 103.06 (C-1); 110.35 (quaternary C of dioxolane); 176.72 (C=O).

### Example 6

Preparation of methyl 6-O-pivaloyl-3,4-O-isopropylidene-β-D-galactopyranoside. Using a procedure similar to that given for example 5, methyl 2-O-benzyl-3,4-O-isopropylidene-β-D-galactopyranoside is prepared. The subsequent reactions for esterification in position 6 with pivaloyl chloride and debenzylation in position 2 are effected respectively with the methods illustrated in examples 3 and 5. There is then obtained, in quantative yield, the methyl 6-O-pivaloyl-3,4-O-isopropylidene-β-D-galactopyranoside as a white solid having the following characteristics: Rf = 0.29 (hexane/AcOEt 4:6); m.p. = 125-129°C (crystallised from petroleum ether; [α]_{D} = +21.4° (c = 1.0, CHCl₃); elemental analysis: (calculated for C₁₅H₂₆O₇: C 56.59%; H 8.23%) C 56.15%; H 8.68%; H 8.23%) C 56.69%; H 8.58%. ¹H-NMR (CDCl₃) δ (ppm): 1.21 (s, 9H, 3CH₃, (CH₃)₃-C); 1.34 and 1.51 (2s, 6H, 2CH₃ of dioxolane); 2.65 (1H, 1OH); 3.54 (s, 3H, OCH₃); 3.51-3.58 (m, 1H, H-5); 4.00-4.13 (m, 4H, H-1, H-2, H-3, H-4); 4.34-4.37 (d, 2H, H-6, H-6'); 4.98 (m, 1H, H-2). ¹³C-NMR (CDCl₃) δ (ppm): 27.03 ((CH₃)₃-C); 26.18 and 27.99 (2CH₃ of dioxolane); 38.67 (CCO); 56.81 (OCH₃); 62.99 (C-6); 70.84-73.53 (C-2, C-4 and C-5); 78.69 (C-3); 103.97 (C-1); 110.35 (quaternary C of dioxolane); 178.09 (C=O).

### Comparative Example: Evaluation of Biological Activity

The biological activity of the compounds described in the present invention was evaluated by examining their capacity to induce erythroid differentiation in the human cell line K562, which is able to differentiate in an erythroid sense - that is to say to express the genes for the γ-globins if subjected to a treatment with modifiers of the biological response adapted for this purpose. The level of differentiation was evaluated by evaluating the positivity of the cells to benzidine. The production of haemoglobin was evaluated by electrophoresis on cellulose acetate and colouration of the gel with a solution based on benzidine-H₂O₂. The expression of genes for γ-globins was evaluated by Northern Blot analysis.

Table 1 shows the results of a comparative study in which the level of erythroid differentiation (expressed as a percentage of the K562 cells which were positive to benzidine with respect to the total cells) was evaluated, obtained following treatment with a human cell line K562 with, independently, GG7B, GG6B and n-butyric acid.

Table 2 shows the results of a second series of experiments in which the cells were treated independently with GG7B, GG6B and GG3B, this latter being previously described in US 5 185 436.

**Table 1**

| Product | Optimal Concentration | Erythroid differentiation (% of K562 cells positive to benzidine) |
|---|---|---|
| Butyric Acid * | 2-5 mM | 15-25% |
| GG7B | 2 mM | 70-75% |
| GG6B | 2 mM | 65% |

**Table 2**

| Product | Optimal Concentration | Erythroid differentiation (% of K562 cells positive to benzidine) |
|---|---|---|
| GG3B | 4-5 mM | 25-30% |
| GG7B | 4-5 mM | >70% |
| GG6B | 4-5 mM | >60% |

| | | |
|---|---|---|
| * Reference compounds | | |

The evaluations were performed after 7 days of induction.

Finally, the following Table 3 shows the results obtained by subjecting the K562 cells to three different combination treatments: a) GG6B at optimum concentration and 25 nM cytosine arabinoside; b) GG7B at optimum concentration and 50 nM cytosine arabinoside; or c) GG7B at sub-optimum concentration and 5µM retinoic acid.

**Table 3**

| Treatment | Erythroid differentiation (% of K562 cells positive to benzidine) |
|---|---|
| a | 80-85% |
| b | 95% |
| c | 67% |

On the basis of the results illustrated in Tables 1, 2 and 3 it is evident that the esters forming the subject of the present invention have a significantly greater biological activity as erythroid differentiation inducers than other previously known inducers utilised hereinbefore, such as n-butyric acid and 3-O-n-butanoyl-1, 2-O-isopropylidene-α-D-glucofuranose.

Moreover, they exhibit low toxicity. These characteristics make them particularly suitable for the preparation of a medicament for the treatment of patients affected by β-talaxemia, making it possible to reduce the need to have recourse to transfusional therapy. These compounds are likewise suitable for the preparation of a medicament for the therapeutic treatment of tumours.

## Claims

1. Compounds which are esters of a carbohydrate, constituted by a carbohydrate unit or a glycoside derived therefrom, having at least one alcohol group esterified with a branched chain carboxylic acid and having the remaining alcohol groups partly protected with a protecting group and partly in the form of free hydroxyls.

2. Compounds according to Claim 1, in which the carbohydrate unit is a monosaccharide unit selected from the group comprising D-glucofuranose, D-galactopyranose and D-mannose, or a disaccharide unit selected from the group comprising saccharose and lactose.

3. Compounds according to Claim 1 or Claim 2, in which the esterifying branched chain carboxylic acids are selected from the group comprising 2-methylpropionic acid or isobutyric acid (HCO₂CH(CH₃)₂), 2,2-dimethylpropionic or pivaloic acid (HCO₂C(CH₃)₃), 3,3-diphenylpropionic acid (HCO₂CH₂CH(C₆H₅)₂), 3-methylbutyric acid (HCO₂CH₂CH(CH₃)₂), 3-phenylbutyric acid (HCO₂CH₂CH(CH₃)C₆H₅), 3,3-dimethylbutyric acid (HCO₂CH₂C(CH₃)₃), 3-methylpentanoic acid (HCO₂CH₂CH(CH₃)CH₂CH₃).

4. Compounds according to Claims 1 to 3 in which the protecting group is isopropylidene.

5. Compounds according to Claims 1 to 4 in which the said protecting group occupies positions 1,2 or 3,4 of the said carbohydrate unit.

6. Compounds according to Claims 1 to 4 in which the said carbohydrate unit is D-glucopyranose or a glycoside derived therefrom esterified on the hydroxyl group in position 3 with a branched chain carboxylic acid selected from the group consisting of 2-methylpropionic acid, 2,2-dimethylpropionic acid, 3,3-diphenylpropionic acid, 3-methylbutyric acid, 3-phenylbutyric acid, 3,3-dimethylbutyric acid, 3-methylpentanoic acid.

7. Compounds according to Claims 1 to 4, in which the said carbohydrate unit is D-galactopyranose or a glycoside derived therefrom esterified on the hydroxyl group in position 2 with a branched chain carboxylic acid selected from the group consisting of 2-methylpropionic acid, 2,2-dimethylpropionic acid, 3,3-diphenylpropionic acid, 3-methylbutyric acid, 3-phenylbutyric acid, 3,3-dimethylbutyric acid, 3-methylpentanoic acid.

8. Compounds according to Claims 1 to 4, in which the said carbohydrate unit is D-galactopyranose or a glycoside derived therefrom esterified on the hydroxyl group in position 6 with a branched chain carboxylic acid selected from the group consisting of 2-methylpropionic acid, 2,2-dimethylpropionic acid, 3,3-diphenylpropionic acid, 3-methylbutyric acid, 3-phenylbutyric acid, 3,3-dimethylbutyric acid, 3-methylpentanoic acid.

9. 3-O-Acyl-1,2-isopropylidene-D-glucofuranose in which the acyl group is derived from a branched chain carboxylic acid selected from the group consisting of 2-methylpropionic acid, 2,2-dimethylpropionic acid, 3,3-diphenylpropionic acid, 3-methylbutyric acid, 3-phenylbutyric acid, 3,3-dimethylbutyric acid, 3-methylpentanoic acid.

10. Methyl-2-O-acyl-3,4-O-isopropylidene-(α or β)-D-galactopyranoside in which the acyl group is derived from a branched chain carboxylic acid selected from the group consisting of 2-methylpropionic acid, 2,2-dimethylpropionic acid, 3,3-diphenylpropionic acid, 3-methylbutyric acid, 3-phenylbutyric acid, 3,3-dimethylbutyric acid, 3-methylpentanoic acid.

11. Methyl-6-O-acyl-3,4-O-isopropylidene-(α or β)-D-galactopyranoside in which the acyl group is derived from a branched chain carboxylic acid selected from the group consisting of 2-methylpropionic acid, 2,2-dimethylpropionic acid, 3,3-diphenylpropionic acid, 3-methylbutyric acid, 3-phenylbutyric acid, 3,3-dimethylbutyric acid, 3-methylpentanoic acid.

12. Compounds according to any of Claims 1 to 11 for use as erythroid cellular differentiation inducers.

13. Compounds according to any of Claims 1 to 11 for use as erythroid cellular differentiation inducers in combination with at least one other modifier of the biological response selected from the group consisting of cytosine arabinoside, retinoic acid, plicamycin, mithramycin, hydroxyurea, guanine, guanosine triphosphate (GTP), guanosine diphosphate (GDP) and guanosine monophosphate (GMP).

14. The use of compounds according to Claims 1 to 11 for the preparation of a medicament for the therapeutic treatment of β-talaxemia.

15. The use of compounds according to Claims 1 to 11 for the preparation of a medicament for the therapeutic treatment of tumours.

16. The use of compounds according to Claims 1 to 11 in combination with at least one other modifier of the biological response selected from the group consisting of cytosine arabinoside, retinoic acid, plicamycin, mithramycin, hydroxyurea, guanine, guanosine triphosphate (GTP), guanosine diphosphate (GDP), and guanosine monophosphate (GMP), for the preparation for a medicament for the therapeutic treatment of β-talaxemia.

17. The use of compounds according to Claims 1 to 11 in combination with at least one other modifier of the biological response selected from the group consisting of cytosine arabinoside, retinoic acid, plicamycin, mithramycin, hydroxyurea, guanine, guanosine triphosphate (GTP), guanosine diphosphate (GDP) and guanosine monophosphate (GMP) for the preparation of a medicament for the therapeutic treatment of tumours.

18. A pharmaceutical composition comprising at least one of the compounds according to Claims 1 to 11 and a pharmaceutically acceptable carrier.

19. A pharmaceutical composition comprising at least one of the compounds according to Claims 1 to 11 in combination with at least one other modifier of the biological response selected from the group consisting of cytosine arabinoside, retinoic acid, plicamycin, mithramycin, hydroxyurea, guanine, guanosine triphosphate (GTP), guanosine diphosphate (GDP) and guanosine monophosphate (GMP) and a pharmaceutically acceptable carrier.
